# EUROPÄISCHE PATENTSCHRIFT

(11) **EP 1 725 609 B1**
(45) Veröffentlichungstag und Bekanntmachung des Hinweises auf die Patenterteilung: **21.05.2008**
(21) Anmeldenummer: 05715729.9
(22) Anmeldetag: 04.03.2005
(51) Int. Cl.: C08K 5/39, C07C 333/00, C07C 333/08, C09J 175/04

(54) **CARBODIIMIDE MIT THIOCARBAMIDS UREESTERGRUPPEN**
CARBODIIMIDES COMPRISING THIOCARBAMIDE ACID ESTER GROUPS
CARBODIIMIDES COMPORTANT DES GROUPES ESTERS DE L'ACIDE THIOCARBAMIQUE

(30) Priorität: 09.03.2004 DE 102004011833
(43) Veröffentlichungstag der Anmeldung: 29.11.2006
(73) Patentinhaber: BASF SE, 67056 Ludwigshafen (DE)
(72) Erfinder: BURGHARDT, Andre, 67240 Bobenheim-Roxheim (DE); HÄBERLE, Karl, 67346 Speyer (DE); LICHT, Ulrike, 68309 Mannheim (DE); SCHUMACHER, Karl-Heinz, 67433 Neustadt (DE)
(86) Internationale Anmeldenummer: PCT/EP2005/002293
(87) Internationale Veröffentlichungsnummer: WO 2005/087718

(56) Entgegenhaltungen:
- EP-A- 0 952 146
- DE-A1- 10 000 656
- US-A- 5 859 166

## Beschreibung

Die Erfindung betrifft Thiocarbamide, enthaltend mindestens eine Carbodiimidgruppe und mindestens eine Thiocarbamidsäureestergruppe der Formel

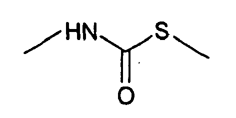

Die Erfindung betrifft auch die Verwendung der Thiocarbamide als Vernetzer oder Stabilisator.

Organische Carbodiimide und deren Verwendung als Zusatzmittel zu wässrigen Polymerdispersionen sind bekannt. Sie werden z.B. Polymerdispersionen zugesetzt, um das Molekulargewicht der Polymere zu erhöhen. Um die Carbodiimide auf einfache Weise homogen in der Dispersion verteilen zu können, werden sie mit hydrophilen Gruppen versehen.

Gemäß DE-A-10 000 656 werden Isocyanatgruppen enthaltende Carbodiimide, mit Hydroxycarbonsäuren umgesetzt. Nachteilig hierbei ist, dass die Umsetzung der Ausgangsverbindungen in wasserfreier Umgebung erfolgen muss, da ansonsten eine unerwünschte Konkurrenz der OH-Gruppen der Hydroxycarbonsäuren und des Wassers um die Isocyanatgruppen erfolgt. Dies führt dazu, dass zur Neutralisation der Carboxylgruppen keine anorganischen Basen verwendet werden können, da sich die erhaltenen Salze nicht im Reaktionsmilieu lösen. Es müssen also Basen verwendet werden, die im Reaktionsmilieu lösliche Salze ergeben; das sind i.A. tertiäre Amine. Die Verwendung tertiärer Amine ist jedoch aus Gründen der Toxikologie und Geruchsbelästigung unerwünscht.

Aus der DE 19 954 500 sind Carbodiimide bekannt, die mit Aminocarbonsäuren hydrophiliert sind.

Diese Verbindungen zeigen z.B. bei der Anwendung als Vernetzer von Klebstoffdispersionen unzureichende Klebeeigenschaften.

Die Aufgabe der Erfindung bestand in der Bereitstellung von Carbodiimide, die zur Vernetzung von Polymerdispersionen geeignet sind, nicht zwangsweise tertiäre Amine enthalten und gegenüber dem Stand der Technik verbesserte Vernetzungseigenschaften aufweisen.

Demgemäß wurden die eingangs definierten Thiocarbamide und ihre Verwendung gefunden.

Die erfindungsgemäßen Thiocarbamide enthalten mindestens eine Carbodiimid- und mindestens eine Thiocarbamidsäureestergruppe.

Carbodiimidgruppen sind in einfacher Weise aus zwei Isocyanatgruppen unter Abspaltung von Kohlendioxid erhältlich:

-R-N=C=O + O=C=N-R

-R-N=C=N-R- + CO₂

Ausgehend von Polyisocyanaten, bzw. Diisocyanaten sind so Carbodiimide mit mehreren Carbodiimidgruppen und gegebenenfalls Isocyanatgruppen, insbesondere endständigen Isocyanatgruppen, erhältlich.

Thiocarbamidsäureestergruppen haben die Formel 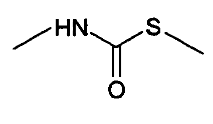 und sind durch Umsetzung von Isocyanatgruppen mit Mercaptogruppen (S-H) erhältlich.

Die erfindungsgemäßen Thiocarbamide haben vorzugsweise einen Gehalt an hydrophilen Gruppen.

Insbesondere sind die Thiocarbamide durch den Gehalt an hydrophilen Gruppen wasserlöslich oder wasserdispergierbar.

Als hydrophile Gruppen in Betracht kommen sowohl ionische Gruppen als auch nichtionische hydrophile Gruppen.

Unter ionischen Gruppen sollen auch Gruppen verstanden werden, die in ionische Gruppen überführbar sind.

Genannt seien insbesondere Säuregruppen, wie Carbonsäure-, Sulfonsäure-, Phosphorsäure-, Phosphonsäuregruppen und deren Salze, d.h. Carboxylate, Sulfonate, Phosphate, Phosphonate.

Nichtionische hydrophile Gruppen sind z.B. Polyalkylenoxidgruppen oder Polyvinylpyrrolidongruppen. Bei den Alkylenoxideinheiten in den Polyalkylenoxidgruppen kann es sich z.B. um Ethylenoxid, Propylenoxid oder Mischungen der beiden handeln. Bevorzugt ist Ethylenoxid.

Für eine ausreichende Hydrophilie sind Polyalkylenoxidgruppen oder Polyvinylpyrrolidongruppen geeignet, die vorzugsweise mindestens 5, besonders bevorzugt mindestens 8 Alkylenoxideinheiten bzw. Vinylpyrrolidoneinheiten enthalten. Die Polyalkylenoxidgruppen oder Polyvinylpyrrolidongruppen können z.B. 5 bis 80 Alkylenoxid- oder Vinylpyrrolidoneinheiten enthalten.

Die Thiocarbamide enthalten bevorzugt 0,01 bis 2 Mol/kg, besonders bevorzugt 0,5 bis 1,8 Mol/kg hydrophile Gruppen, bezogen auf das Gewicht der Thiocarbamide.

Der Gewichtsanteil der hydrophilen Gruppen, insbesondere der nicht-ionischen hydrophilen Gruppen liegt im allgemeinen zwischen 1 bis 30 Gew.-%, bezogen auf die Thiocarbamide.

Der Anteil an Carbodiimid-Gruppen beträgt im allgemeinen 0,05 bis 8, bevorzugt 0,10 bis 5 mol/kg bezogen auf das Gewicht der Thiocarbamide.

Die Carbodiimideinheiten in den erfindungsgemäßen Thiocarbamide werden im wesentlichen in der Weise gebildet, daß je 2 NCO-Gruppen unter Abspaltung von Kohlendioxid zu einer Carbodiimideinheiten zusammentreten.

Die Thiocarbamide enthalten bevorzugt mindestens eine Carbodiimideinheit, bevorzugt mehr als eine Carbodiimideinheiten, besonders bevorzugt beträgt der mittlere Kondensationsgrad (Zahlenmittelwert), d.h. die mittlere Anzahl an Carbodiimideinheiten in den erfindungsgemäßen Carbodiimiden 1 bis 20, insbesondere 2 bis 15.

Die erfindungsgemäßen Thiocarbamide sind vorzugsweise erhältlich durch Umsetzung von
a) Carbodiimide mit mindestens einer Isocyanatgruppe,
b) Mercaptoverbindungen mit mindestens einer Mercaptogruppe und
c) gegebenenfalls weiteren Verbindungen mit Isocyanatgruppen oder mit Isocyanat reaktiven Gruppen.

Die Carbodiimide a) sind wie oben beschrieben aus Polyisocyanaten erhältlich. Als Polyisocyanate in Betracht kommen insbesondere Diisocyanate X(NCO)₂, wobei X für einen ali-phatischen Kohlenwasserstoffrest mit 4 bis 12 Kohlenstoffatomen, einen cycloaliphatischen oder aromatischen Kohlenwasserstoffrest mit 6 bis 15 Kohlenstoffatomen oder einen araliphatischen Kohlenwasserstoffrest mit 7 bis 15 Kohlenstoffatomen steht. Beispiele derartiger Diisocyanate sind Tetramethylendiisocyanat, Hexamethylendiisocyanat, Dodecamethylendiisocyanat, 1,4-Diisocyanatocyclohexan, 1-Isocyanato-3,5,5-trimethyl-5-isocyanatomethylcyclohexan (IPDI), 2,2-Bis-(4-isocyanatocyclohexyl)-propan, Trimethylhexandiisocyanat, 1,4-Diisocyanatobenzol, 2,4-Diisocyanatotoluol, 2,6-Diisocyanatotoluol, 4,4'-Diisocyanato-diphenylmethan, 2,4'-Diisocyanato-diphenylmethan, p-Xylylendiisocyanat, Tetramethylxylylendiisocyanat (TMXDI), die Isomeren des Bis-(4-isocyanatocyclohexyl)methans (HMDI) wie das trans/trans-, das cis/cis- und das cis/trans-Isomere sowie aus diesen Verbindungen bestehende Gemische.

Bevorzugt sind aliphatische oder araliphatische C₄ bis C₂₀ Polyisocyanate bzw. Diisocyanate.

Besonders bevorzugt ist TMXDI.

Bei den Mercaptoverbindungen b) handelt es sich um Verbindungen mit mindestens einer Mercaptogruppe, vorzugsweise mit ein oder zwei Mercaptogruppen, besonders bevorzugt mit einer Mercaptogruppe.

Bei den Verbindungen b) handelt es sich vorzugsweise um niedermolekulare Verbindungen mit einem Molgewicht von 90 bis 1000, insbesondere von 90 bis 500 g/mol.

Die Mercaptoverbindungen haben vorzugsweise zusätzlich mindestens eine, insbesondere ein oder zwei, besonders bevorzugt eine hydrophile Gruppe.

Insbesondere haben demgemäß die Thiocarbamide durch Mercaptoverbindungen b) den oben angegebenen Gehalt an hydrophilen Gruppen. Bei den hydrophilen Gruppen in den Mercaptoverbindungen handelt es sich daher um die oben aufgeführten ionischen oder hydrophilen nicht-ionischen Gruppen.

Bevorzugte Mercaptoverbindungen sind Mercaptosäuren wie Mercaptocarbon-, sulfon- oder phosphonsäuren, besonders bevorzugt Mercaptocarbon- oder sulfonsäuren.

Als Beispiele hierfür sind zu nennen:

Mercaptoessigsäure, 2- und 3-Mercaptopropionsäure, Mercaptobernsteinsäure, Mercaptoethansulfonsäure, 2- und 3-Mercaptopropansulfonsäure.

Mercaptoverbindungen mit kationischen Gruppen sind z.B. Mercaptoamine mit tertiären Aminogruppen, z.B. Dialkylaminothiole wie 2-Dimethylamino-1-ethanthiol, 3-Diethylamino-1-Propanthiol etc.

Mercaptoverbindungen mit nicht-ionischen hydrophilen Gruppen sind z.B. Mercaptogruppen tragende Polyalkylenoxide oder Polyvinylpyrrolidone. Die Mercaptoverbindungen können neben Mercaptogruppen und hydrophilen Gruppen weitere mit Isocyanat reaktionsfähige Gruppen tragen, z.B. Hydroxylgruppen oder Aminogruppen. Genannt seien z.B. Cystein oder Homocystein.

Insbesondere handelt es sich bei den Mercaptoverbindungen b) um aliphatische Verbindungen.

Verbindungen c) können beliebige Polyisocyanate ohne Carbodiimidgruppen oder Verbindungen mit gegenüber Isocyanat reaktiven Gruppen sein.

Als Polyisocyanate kommen neben den oben aufgeführten Polyisocyanaten auch aromatische Polyisocyanate und Polyisocyanate mit mehr als zwei Isocyanatgruppen in Betracht.

Verbindungen mit gegenüber Isocyanat reaktiven Gruppen sind z.B. Hydroxylverbindungen oder Aminoverbindungen, z.B. Alkandiole, aliphatische Diamine, Alkanolamine etc.

Im allgemeinen beträgt der Anteil der Komponenten (c), bezogen auf den Anteil aller Komponenten (a) bis (c), die zur Herstellung der Thiocarbamide eingesetzt werden, nicht mehr als 0 bis 40, bevorzugt 0 bis 30 Gew.-%, besonders bevorzugt weniger als 10 Gew.-%, insbesondere weniger als 5 Gew.-%.

Ein Gehalt an Komponente c) wird für die Zwecke der vorliegenden Erfindung nicht zwingend benötigt, auf Komponente c) kann daher ganz verzichtet werden.

Die Herstellung der erfindungsgemäßen Thiocarbamide erfolgt im wesentlichen durch zwei Umsetzungsschritte, indem man
I. Carbodiimide mit endständigen Isocyanatgruppen herstellt, indem man einen Teil der Isocyanatgruppen der Komponente (a) carbodimidisiert und
II. die gemäß Schritt I hergestellten Verbindungen mit endständigen Isocyanatgruppen mit der Komponente (b) und gegebenenfalls den Komponenten (c) umsetzt.

In Schritt I werden durch allgemein bekannte Umsetzung der Isocyanatgruppen miteinander unter Abspaltung von Kohlendioxid in Gegenwart von üblichen Katalysatoren, die für diese Umsetzung bekannt sind, Carbodiimidstrukturen erzeugt. In Schritt II werden die Carbodiimide und gegebenenfalls sonstige Polyisocyanate mit gegenüber Isocyanaten reaktiven Verbindungen zur Herstellung von Thiocarbamidsäureesterstrukturen und gegebenenfalls Urethan- und/oder Hamstoffstrukturen in bekannter Weise umgesetzt.

Das molare Verhältnis der NCO-Gruppen zu der Summe aus den gegenüber Isocyanaten reaktiven Gruppen beträgt üblicherweise 10:1 bis 0,2:1, bevorzugt 5:1 bis 0,5:1.

Alternativ können die erfindungsgemäßen Carbodiimide dadurch erhalten werden, daß man zunächst Polyisocyanate mit den Komponenten (b) und gegebenfalls (c) umsetzt, wobei das Verhältnis von eingesetzten Isocyanatgruppen zu der Summe aus den gegenüber Isocyanaten reaktiven Gruppen mindestens 2 : 1 beträgt, und anschließend das Isocyanatgruppen aufweisende Reaktionsprodukt in Gegenwart von Katalysatoren unter Kohlendioxidfreisetzung zu Carbodiimide umsetzt. Nach dieser Verfahrensvariante werden zunächst bis zu 50 Gew.-%, vorzugsweise bis zu 25 Gew.-% der Isocyanatgruppen der Polyisocyanate mit den gegenüber Isocyanaten reaktiven Verbindungen umgesetzt und danach die freien Isocyanatgruppen in Gegenwart von Katalysatoren unter Kohlendioxidfreisetzung ganz oder teilweise zu Carbodiimidgruppen umgesetzt.

Die Umsetzungen können bevorzugt in Gegenwart eines Lösungsmittels durchgeführt werden. Als Lösungsmittel sind vor allem solche Verbindungen geeignet, die das Reaktionsprodukt der Umsetzung gemäß Schritt I gut lösen und außerdem mit Wasser mischbar sind, beispielsweise Propanon, Tetrahydrofuran, Dioxan, N-Methylpyrrolidon, Dimethylformamid, Dimethylacetamid und/oder Propylencarbonat. Bevorzugt werden Lösemittel mit einem Siedepunkt bei 1013 mbar von weniger als 100 °C verwendet.

Der Verfahrensschritt, in dem die Carbodiimidgruppen gebildet werden, kann bei erhöhten Temperaturen, z.B. bei Temperaturen von 50 bis 200°C, vorzugsweise von 150 bis 185°C, zweckmäßigerweise in Gegenwart von Katalysatoren durchgeführt werden. Als Katalysatoren vorzüglich bewährt haben sich z.B. Phosphorverbindungen, die vorzugsweise ausgewählt werden aus der Gruppe der Phospholene, Phospholenoxide, Phospholidine und Phospholinoxide. Wenn die Reaktionsmischung den gewünschten Gehalt an NCO-Gruppen besitzt, wird die Polycarbodiimidbildung üblicherweise beendet. Hierzu können die Katalysatoren unter vermindertem Druck abdestilliert oder durch Zusatz eines Desaktivators, wie z.B. Phosphortrichlorid, desaktiviert werden. Die Katalysatoren können auch im Produkt verbleiben. Die Polycarbodiimidherstellung kann ferner in Abwesenheit oder Gegenwart von unter den Reaktionsbedingungen inerten Lösungsmitteln durchgeführt werden.

Wird Polyisocyanat zuerst zu einem Isocyanatgruppen aufweisenden Carbodiimid (Schritt I) umgesetzt, so weist das in Schritt I gebildete Zwischenprodukt bevorzugt einen NCO-Gehalt von 1 bis 18 Gew. % auf.

Durch geeignete Wahl der Reaktionsbedingungen wie z.B. der Reaktionstemperatur, der Katalysatorart und der Katalysatormenge sowie der Reaktionszeit kann der Fachmann in der üblichen Weise den Kondensationsgrad einstellen. Der Verlauf der Reaktion kann am einfachsten durch Bestimmung des NCO-Gehaltes verfolgt werden. Auch andere Parameter wie z.B. Viskositätsanstieg, Farbvertiefung oder CO₂-Entwicklung kann man für die Verfolgung des Ablaufs und die Steuerung der Reaktion heranziehen.

Die Temperatur bei dem Schritt, in dem die Thiocarbamidsäureestergruppen und gegebenenfalls Urethan- und Harnstoffgruppen gebildet werden, beträgt üblicherweise 10 bis 100 °C.

Die erfindungsgemäßen Thiocarbamide eignen sich vor allem zur Molekulargewichtserhöhung von Polymeren, die in Form einer wässrigen Dispersion oder Lösung vorliegen. Bevorzugt sind wässrige Dispersionen.

Als Polymer, kommen praktisch alle filmbildenden Polymerisate in Betracht.

In einer bevorzugten Ausführungsform tragen die Polymere Carboxylgruppen, im allgemeinen in Mengen von 0,01 bis 2 mol/kg Polymer, vorzugsweise 0,05 bis 2 mol/kg.

Geeignete Polymere sind beispielsweise wasserdispergierbare Polyurethane. Derartige Polyurethane und die diese enthaltenen Dispersionen sind allgemein bekannt.

Bevorzugt sind derartige Polyurethane aufgebaut aus
IIa) Diisocyanaten mit 4 bis 30 C-Atomen,
IIb) Diolen, von denen
   IIb1) 10 bis 100 mol-%, bezogen auf die Gesamtmenge der Diole (IIb), ein Molekulargewicht von 500 bis 5000 aufweisen, und
   IIb2) 0 bis 90 mol-%, bezogen auf die Gesamtmenge der Diole, ein Molekulargewicht von 60 bis 500 g/mol aufweisen,
IIc) von den Monomeren (IIa) und (IIb) verschiedene Monomere mit wenigstens einer Isocyanatgruppe oder wenigstens einer gegenüber Isocyanatgruppen reaktiven Gruppe, die darüberhinaus wenigstens eine hydrophile Gruppe oder eine potentiell hydrophile Gruppe tragen, wodurch die Wasserdispergierbarkeit der Polyurethane bewirkt wird,
IId) gegebenenfalls weiteren von den Monomeren (IIa) bis (IIc) verschiedenen mehrwertigen Verbindungen mit reaktiven Gruppen, bei denen es sich um alkoholische Hydroxylgruppen, primäre oder sekundäre Aminogruppen oder Isocyanatgruppen handelt und
IIe) gegebenenfalls von den Monomeren (IIa) bis (IId) verschiedenen einwertigen Verbindungen mit einer reaktiven Gruppe, bei der es sich um eine alkoholische Hydroxylgruppe, eine primäre oder sekundäre Aminogruppe oder eine Isocyanatgruppe handelt.

Als Monomere (IIa) kommen die üblicherweise in der Polyurethanchemie eingesetzten aliphatischen oder aromatischen Diisocyanate in Betracht. Bevorzugt sind die Monomere (IIa) oder deren Mischungen, die auch als Monomere (IIa) in der DE-A-19521500 erwähnt sind.

Als Monomere (IIb) und (IId) kommen bevorzugt die in der DE-A-19521500 als Monomere (IIb) und (IId) genannten in Betracht.

Monomere IIb1 sind beispielsweise Polyester- oder Polyetherdiole.

Bei den Monomeren IIb2 handelt es sich beispielsweise um aliphatische Diole mit 2 bis 12 C-Atomen, z.B. 1,4-Butandiol oder 1,6-Hexandiol.

Als Monomere (IId) sind beispielsweise aliphatische Amine mit 2 bis 12 C-Atomen und 2 bis 4 Gruppen, ausgewählt aus der Gruppe der primären oder sekundären Aminogruppen, geeignet. Beispiele sind Ethylendiamin, Isophorondiamin oder Diethylentriamin.

Um die Wasserdispergierbarkeit der Polyurethane zu erreichen, sind die Polyurethane neben den Komponenten (IIa), (IIb) und (IId) aus von den Komponenten (IIa), (IIb) und (IId) verschiedenen Monomere (IIc), die wenigstens eine Isocyanatgruppe oder wenigstens eine gegenüber Isocyanatgruppen reaktive Gruppe und darüberhinaus wenigstens eine hydrophile Gruppe oder eine Gruppe, die sich in eine hydrophile Gruppe überführen läßt, tragen, aufgebaut. Im folgenden Text wird der Begriff "hydrophile Gruppen oder potentiell hydrophile Gruppen" mit "(potentiell) hydrophile Gruppen" abgekürzt. Die (potentiell) hydrophilen Gruppen reagieren mit Isocyanaten wesentlich langsamer als die funktionellen Gruppen der Monomere, die zum Aufbau der Polymerhauptkette dienen.

Bevorzugte Monomere (IIc) sind ebenfalls die in der DE-A-19521500 als Monomere (IIc) bezeichneten.

Der Anteil der Komponenten mit (potentiell) hydrophilen Gruppen an der Gesamtmenge der Komponenten (IIa), (IIb), (IIc), (IId) und (IIe) wird im allgemeinen so bemessen, daß die Molmenge der (potentiell) hydrophilen Gruppen, bezogen auf die Gewichtsmenge aller Monomere (a) bis (e), 80 bis 1200, bevorzugt 100 bis 1000 und besonders bevorzugt 150 bis 800 mmol/kg beträgt.

Bei den (potentiell) hydrophilen Gruppen kann es sich um nichtionische, z.B. Polyethylenoxidgruppen oder bevorzugt um (potentiell) ionische hydrophile Gruppen, z.B. Carboxylat- oder Sulfonat-Gruppen handeln. Bevorzugt wird ohne wirksame Mengen an nichtionischen Gruppen gearbeitet.

Der Gehalt an nichtionischen hydrophilen Gruppen, falls solche eingebaut werden, beträgt im allgemeinen bis 5, bevorzugt bis 3, besonders bevorzugt bis 1 Gew.-%, bezogen auf die Gewichtsmenge aller Monomere (IIa) bis (IIe).

Monomere (IIe), die gegebenenfalls mitverwendet werden, sind Monoisocyanate, Monoalkohole und mono-primäre und -sekundäre Amine. Im allgemeinen beträgt ihr Anteil maximal 10 mol-%, bezogen auf die gesamte Molmenge der Monomere. Diese monofunktionellen Verbindungen tragen üblicherweise weitere funktionelle Gruppen wie Carbonylgruppen und dienen zur Einführung von funktionellen Gruppen in das Polyurethan, die die Dispergierung bzw. die Vernetzung oder weitere polymeranaloge Umsetzung des Polyurethans ermöglichen.

Auf dem Gebiet der Polyurethanchemie ist allgemein bekannt, wie das Molekulargewicht der Polyurethane durch Wahl der Anteile der miteinander reaktiven Monomere sowie dem arithmetischen Mittel der Zahl der reaktiven funktionellen Gruppen pro Molekül eingestellt werden kann.

Normalerweise werden die Komponenten (IIa) bis (IIe) sowie ihre jeweiligen Molmengen so gewählt, daß das Verhältnis A : B mit
A) der Molmenge an Isocyanatgruppen und
B) der Summe aus der Molmenge der Hydroxylgruppen und der Molmenge der funktionellen Gruppen, die mit Isocyanaten in einer Additionsreaktion reagieren können

0,5 : 1 bis 2 : 1, bevorzugt 0,8 : 1 bis 1,5, besonders bevorzugt 0,9 : 1 bis 1,2 : 1 beträgt. Ganz besonders bevorzugt liegt das Verhältnis A : B möglichst nahe an 1 : 1.

Die eingesetzten Monomere (IIa) bis (IIe) tragen im Mittel üblicherweise 1,5 bis 2,5, bevorzugt 1,9 bis 2,1, besonders bevorzugt 2,0 Isocyanatgruppen bzw. funktionelle Gruppen, die mit Isocyanaten in einer Additionsreaktion reagieren können.

Die verschiedenen Herstellmethoden der Polyurethane sind allgemein bekannt und beispielsweise in der DE-A-19807754 näher beschrieben.

Weiterhin kann es sich bei den Polymeren um solche handeln, die durch radikalische Polymerisation von ethylenisch ungesättigten Verbindungen (Monomeren) erhältlich sind (kurz Polyaddukt).

Derartige Polyaddukte sind im allgemeinen aufgebaut aus
IIIa) 30 bis 100 Gew.-% Hauptmonomere ausgewählt aus C₁- bis C₂₀-Alkyl(meth)acrylaten, Vinylestern von bis zu 20 C-Atome enthaltenden Carbonsäuren, Vinylaromaten mit bis zu 20 C-Atomen, ethylenisch ungesättigten Nitrilen, Vinylhalogeniden und aliphatischen Kohlenwasserstoffen mit 2 bis 8 C-Atomen und 1 oder 2 Doppelbindungen,
IIIb) 0 bis 20, bevorzugt 0,01 bis 20 Gew.-% einer Carbonsäure mit einer olefinischen Doppelbindung und
IIIc) 0 bis 20 Gew.-% von (IIIa) und (IIIb) verschiedenen radikalisch polymerisierbaren Monomeren.

Als Monomere (IIIa) zu nennen sind z.B. (Meth)acrylsäurealkylester mit einem C₁-C₁₀-Alkylrest, wie Methylmethacrylat, Methylacrylat, n-Butylacrylat, Ethylacrylat und 2-Ethylhexylacrylat.

Insbesondere sind auch Mischungen der (Meth)acrylsäurealkylester geeignet.

Vinylester von Carbonsäuren mit 1 bis 20 C-Atomen sind z.B. Vinyllaurat, -stearat, Vinylpropionat und Vinylacetat.

Als vinylaromatische Verbindungen kommen Vinyltoluol, alpha- und p-Methylstyrol, alpha-Butylstyrol, 4-n-Butylstyrol, 4-n-Decylstyrol und vorzugsweise Styrol in Betracht.

Beispiele für Nitrile sind Acrylnitril und Methacrylnitril.

Die Vinylhalogenide sind mit Chlor, Fluor oder Brom substituierte ethylenisch ungesättigte Verbindungen, bevorzugt Vinylchlorid und Vinylidenchlorid.

Als nicht aromatische Kohlenwasserstoffe mit 2 bis 8 C-Atomen und ein oder zwei olefinischen Doppelbindungen seien Butadien, Isopren, und Chloropren, sowie Ethylen, Propylen und Isobutylen genannt.

Die Monomeren (IIIa) werden auch vorzugsweise im Gemisch eingesetzt.

Vinylaromatische Verbindungen wie Styrol werden z.B. häufig im Gemisch mit C₁-C₂₀-Alkyl(meth)acrylaten, insbesondere mit C₁-C₈-Alkyl(meth)acrylaten, oder nicht aromatischen Kohlenwasserstoffen wie Isopren oder vorzugsweise Butadien eingesetzt.

Als Monomere (IIIb) kommen vorzugsweise (Meth)acrylsäure oder Maleinsäure in Betracht.

Als Monomere (IIIc) kommen z.B. in Betracht: Ester der Acryl- und Methacrylsäure von Alkoholen mit 1 bis 20 C-Atomen, die außer dem Sauerstoffatom in der Alkoholgruppe mindestens ein weiteres Heteroatom enthalten und/oder die einen aliphatischen oder aromatischen Ring enthalten, wie 2-Ethoxyethylacrylat, 2-Butoxyethyl(meth)acrylat, Dimethylaminoethyl(meth)acrylat, Diethylaminoethyl(meth)acrylat, (Meth)acrylsäurearyl-, -alkaryl- oder Cycloalkylester, wie Cyclohexyl(meth)acrylat, Phenylethyl-(meth)acrylat, Phenylpropyl-(meth)acrylat oder Acrylsäureester von heterocyclischen Alkoholen wie Furfuryl(meth)acrylat genannt.

Darüber hinaus kommen als Monomer (IIIc) noch Monomere mit Amino- oder Amidgruppen wie (Meth)acrylamid, sowie deren am Stickstoff mit C₁-C₄-Alkyl substituierten Derivate, in Betracht.

Von Bedeutung als Monomere (IIIc) sind insbesondere hydroxyfunktionelle Monomere, z.B. (Meth)acrylsäure-C₁-C₁₅-alkylester, welche durch ein oder zwei Hydroxygruppen substituiert sind. Insbesondere von Bedeutung als hydroxyfunktionelle Comonomere sind (Meth)acrylsäure-C₂-C₈- hydroxyalkylester, wie n-Hydroxyethyl-, n-Hydroxypropyl- oder n-Hydroxybutyl(meth)acrylat.

Die Herstellung des Polymers (PIII) erfolgt durch radikalische Polymerisation. Geeignete Polymerisationsmethoden, wie Substanz-, Lösungs-, Suspensions- oder Emulsionspolymerisation sind dem Fachmann bekannt.

Vorzugsweise wird das Copolymerisat durch Lösungspolymerisation mit anschließender Dispergierung in Wasser oder besonders bevorzugt durch Emulsionspolymerisation hergestellt.

Besonders bevorzugt handelt es sich daher um ein Emulsionspolymerisat.

Die Comonomeren können bei der Emulsionspolymerisation wie üblich in Gegenwart eines wasserlöslichen Initiators und eines Emulgators bei vorzugsweise 30 bis 95°C polymerisiert werden.

Geeignete Initiatoren sind z.B. Natrium-, Kalium- und Ammoniumpersulfat, tert.-Butylhydroperoxide, wasserlösliche Azoverbindungen oder auch Redoxinitiatoren.

Als Emulgatoren dienen z.B. Alkalisalze von längerkettigen Fettsäuren, Alkylsulfate, Alkylsulfonate, alkylierte Arylsulfonate oder alkylierte Biphenylethersulfonate. Des weiteren kommen als Emulgatoren Umsetzungsprodukte von Alkylenoxiden, insbesondere Ethylen- oder Propylenoxid mit Fettalkoholen, -säuren oder Phenolen, bzw. Alkylphenolen in Betracht.

Im Falle von wäßrigen Sekundärdispersionen wird das Copolymerisat zunächst durch Lösungspolymerisation in einem organischen Lösungsmittel hergestellt und anschließend unter Zugabe von Salzbildnern, z.B. von Ammoniak, zu Carbonsäuregruppen enthaltenden Copolymerisaten in Wasser ohne Verwendung eines Emulgators oder Dispergierhilfsmittels dispergiert. Das organische Lösungsmittel kann abdestilliert werden. Die Herstellung von wäßrigen Sekundär-dispersionen ist dem Fachmann bekannt und z.B. in der DE-A-37 20 860 beschrieben.

Zur Einstellung des Molekulargewichts können bei der Regler eingesetzt werden. Geeignet sind z.B. -SH enthaltende Verbindungen wie Mercaptoethanol, Mercaptopropanol, Thiophenol, Thioglycerin, Thioglykolsäureethylester, Thioglykolsäuremethylester und tert.-Dodecylmercaptan, sie können z.B. in Mengen von 0 bis 0,5 Gew.-%, bezogen auf das Copolymerisat, zusätzlich eingesetzt werden.

Die Art und Menge der Comonomeren wird vorzugsweise so gewählt, daß das erhaltene Copolymerisat eine Glasübergangstemperatur von -60 bis +140°C, vorzugsweise - 60 bis +100°C aufweist. Die Glasübergangstemperatur des Copolymerisats wird durch Differentialthermoanalyse oder Differential Scanning Calorimetrie nach ASTM 3418/82, bestimmt.

Weiterhin kann es sich bei den Polymeren um um einen wasserdispergierbaren Polyester, der Carboxylgruppen trägt, handeln.

Die wasserdispergierbaren Polyester, die Carboxylgruppen tragen, sind beispielsweise aus Encyclopedia of Polymer Science and Engineering, John Wiley & Sons, second edition, volume 12, Seiten 300 bis 313 bekannt.

Die wässrigen Dispersionen oder Lösungen, die das Polymer enthalten, weisen üblicherweise einen Feststoffgehalt von 10 bis 70 Gew.-% auf.

Mischungen aus Thiocarbamiden und wässrigen Dispersionen bzw. Lösungen des Polymeren enthalten Thiocarbamide und Polymer bevorzugt im Gewichtsverhältnis 0,005 : 1 bis 1 : 1.

Die Zugabe der Thiocarbamide zu Polymerdispersionen oder Lösungen ist unkritisch und kann beispielsweise so vorgenommen werden, daß man in die wässrige Dispersionen enthaltend Polymer einrührt. Die Zugabe kann zu einem beliebigen Zeitpunkt vor deren Anwendung erfolgen. Die erhaltenen Mischungen sind lagerstabil.

Die erfindungsgemäßen Mischungen aus Thiocarbamiden und Polymer können handelsübliche Hilfs- und Zusatzstoffe wie Netzmittel, Entschäumer, Mattierungsmittel, Emulgatoren, Verdickungsmittel und Thixotropiermittel, Farbmittel wie Farbstoffe und Pigmente enthalten.

Sie eignen sich beispielsweise zum Verkleben oder Beschichten unterschiedlicher Substrate wie Holz, Metall, Kunststoff, Papier, Leder oder Textil, für die Imprägnierung von Textilien sowie für die Herstellung von Formkörpern und Druckfarben.

Die Verarbeitung der erfindungsgemäßen Dispersionen kann dabei nach den in der Klebstoff, Leder- oder Lackindustrie allgemein üblichen Verfahren erfolgen, also indem man die Dispersionen auf das Substrat sprüht, walzt oder rakelt und anschließend trocknet.

Für den Fall der Verarbeitung als Klebstoff werden die beschichteten Werkstücke entweder vor dem Trocknen des Dispersionsfilms oder nach dem Trocknen mit einem anderen Werkstück bevorzugt unter Anwendung von Druck zusammengefügt.

Besonders feste Klebstoffverbunde erhält man, wenn man Werkstücke, die mit einem getrockneten Klebstofffilm versehen sind, unmittelbar vor, während oder nach dem Zusammenfügen auf eine Temperatur von ca. 50 bis 100°C erwärmt.

Die nach diesen Methoden hergestellten Klebstoffverbunde zeichnen sich insbesondere dadurch aus, daß sie lagerbeständig sind und eine hohe Wärmestandfestigkeit haben.

Weiterhin lassen sich die Thiocarbamide zur Herstellung von Klebstofffolien einsetzen. Hierzu werden wässrige Dispersionen, enthaltend z.B. ein Polyurethan oder Polyaddukt ein mit Thiocarbamiden und gegebenenfalls weiteren Hilfsstoffen abgemischt. Diese Mischung wird nach den üblichen vorgenannten Methoden auf Kunststoffolien aufgetragen, wobei coronabehandelte Polyethylenfolie bevorzugt ist. Die Auftragsmengen betragen üblicherweise 5 bis 6 g/m².

Beschichtete Klebstofffolie, z.B. aus coronabehandelter Polyethylenfolie, eignen sich zum Bekleben von Gegenständen aller Art. Wird ein Polymer eingesetzt, das sich als Haftklebstoff eignet, so zeichnet sich die beschichtete Folie insbesondere dadurch aus, daß sie sich rückstandsfrei vom Substrat lösen läßt.

Derartige Klebstofffolien eignen sich deshalb insbesondere zur Herstellung von Etiketten oder für die Verwendung als Schutzfolien, um Gegenstände, insbesondere solche mit empfindlichen Oberflächen wie lackierte Oberflächen oder solche aus Plexiglas, Polycarbonat oder Glas, z.B. Bildschirme oder Scheiben, bei der Lagerung und Transport vor mechanischen Beschädigungen, z.B. Verkratzen, oder sonstigen Umwelteinflüssen zu schützen. Sie besitzen zusätzlich den Vorteil, daß sie einen guten "Tack" aufweisen, d.h. daß die Folie bei bloßer Berührung ohne Anwendung von hohem Druck, z.B. durch Aufstreichen mit der Hand oder durch Auflegen der Folie, bereits auf dem Substrat haftet und sich mit mäßiger Kraft (z.B. unter Anwendung von 1,25 bis 2,5 N bei einem Klebstoffstreifen mit einer Breite von 25 mm) wieder vom Substrat abschälen lassen.

Die Thiocarbamide eignen sich weiterhin auch als Stabilisator für Ester- oder amidgruppenhaltige Polymere.

Vorteilhaft ist insbesondere die Lagerstabilität der Thiocarbamide enthaltenden Polymerdispersionen oder-Lösungen und gute anwendungstechnische Eigenschaften bei der Verwendung als Klebstoff, z.B. eine hohe Schälfestigkeit und gute Wärmestandfestigkeit.

### Experimenteller Teil

1. Hydrophile Carbodiimide
   1.1. Herstellung eines hydrophilen Carbodiimids mit ThiocarbamidsäureesterGruppen (erfindungsgemäß)
      471 g eines NCO-terminierten Carbodiimids aus TMXDI mit einem NCO-Gehalt von 7,8 Gew.% wurden in 800 g Aceton gelöst und auf 45°C erwärmt. Hierzu wurde unter Rühren eine Mischung von 40 g NaOH, 73,7 g Mercaptoessigsäure, 320 g Wasser und 200 g Aceton gegeben. Nach 6 min Rühren wurde mit 700 g Wasser verdünnt und das Aceton im Vakuum abgezogen.
      Man erhielt eine kolloidale, wässrige Lösung eines Carbodiimids mit 36,4% Festgehalt, einem pH-Wert von 10,8 und einem LD-Wert von 100.
   1.2. Herstellung eines hydrophilen Carbodiimids mit Harnstoff-Gruppen (Vergleich, nach der Lehre der DE 19 954 500)
      471 g eines NCO-terminierten Carbodiimids aus TMXDI mit einem NCO-Gehalt von 7,8 Gew.% wurden in 800 g Aceton gelöst und auf 45°C erwärmt. Hierzu wurde unter Rühren eine Mischung von 40 g NaOH, 60,0 g Glycin, 320 g Wasser und 200 g Aceton gegeben. Nach 6 min Rühren wurde mit 700 g Wasser verdünnt und das Aceton im Vakuum abgezogen.
      Man erhielt eine kolloidale, wässrige Lösung eines Carbodiimids mit 36,8% Festgehalt, einem pH-Wert von 12,7 und einem LD-Wert von 100.
2. Herstellung einer Polyurethan-Dispersion
   Abkürzungen:
   DMPA: Dimethylolpropionsäure
   DBTL: Dibutylzinndilaurat
   IPDI: Isophorondiisocyanat

   23,8 kg (9,6 mol) eines Polyesterols aus Adipinsäure und Butandiol-1,4 der OH-Zahl 45,2, 0,429 kg (3,2 mol) DMPA, 0,008 kg DBTL und 3,2 kg Aceton wurden in einem Rührkessel auf 60°C aufgeheizt. Dazu wurden 3,59 kg (16,2 mol) IPDI gegeben und 3 Stunden bei 90°C reagieren lassen. Dann wurde mit 29 kg Aceton verdünnt und dabei die Temperatur auf 30°C gesenkt. Anschließend wurden 0,23 kg (2,88 mol) NaOH gelöst in 0,23 kg Wasser zugegeben. Fünf Minuten danach wurden 0,16 kg einer 50 gew.%igen Lösung eines NCO-terminierten Carbodiimids aus TMXDI mit einem NCO-Gehalt von 7,8 Gew.% in Aceton und anschließend 1,2 kg einer 50 gew.%igen wässrigen Lösung des Natriumsalzes der 2'-Aminoethyl-2-Aminoethansulfonsäure zugegeben. Vier Minuten danach wurde mit 42 kg Wasser dispergiert und dann das Aceton im Vakuum abgezogen.
   Man erhielt eine wässrige Polyurethan-Dispersion mit einem Festgehalt von 39,2%, einem pH-Wert von 7,9 und einer Viskosität bei 250 s⁻¹ von 160 mPas.
3. Prüfung als Thermokaschierkleber
   Zur Prüfung als Thermokaschierkleber wurden 100 Teile der Polyurethan-Dispersion mit 100 Teilen Airflex EP 17 (eine Dispersion eines Copolymeren aus Vinylacetat und Ethylen) und 10 Teilen des Vernetzers aus Beispiel 1.1. und 1.2. gemischt. Die Mischung wurde auf ein Kfz-Formteil aus ABS aufgetragen und 1 h bei 23°C getrocknet. Nach der Trocknung wird in einer beheizten Presse eine für die technische Automobilkaschierung handelsübliche PVC-Folie bei 60°C Filmtemperatur und 1 bar aufkaschiert.
   Die verklebten Formteile aus ABS und PVC-Folie sind 50 mm breit und 150 mm lang, wobei die Verklebung auf einer Länge von etwa 12 cm vorgenommen wird und 3 cm Folie unverklebt bleiben. Sie wurden fünf Tage lang bei 23°C gelagert. Dann wurden die Schälfestigkeiten bei 100°C gemessen.
   Beschreibung der Prüfmethoden:
   a) Schälfestigkeit:
      Rollenschälversuch bei 100°C mittels einer üblichen Zugmaschine, Abzugswinkel 90°, Abzugsgeschwindigkeit 100 mm/min. Gemessen wird die Abzugskraft in N/50 mm.
   b) Wärmestandfestigkeit:
      Die zu prüfende Kaschierung aus der PVC-Folie und dem ABS-Formteil wird waagrecht mit der PVC-Folie nach unten in einen Rahmen eingespannt und an dem freien, nicht verklebten PVC-Ende wird ein Gewicht von 300 g angebracht, sodaß die Folie unter dem Gewicht bis zur Verklebungsstelle auf dem ABS senkrecht nach unten abknickt. Man erwärmt den Prüfkörper auf 80°C und misst die Strecke, die sich die Folie ausgehend vom Beginn der Verklebung von dem ABS gelöst hat (Angabe in mm).
      Schälfestigkeit:
      Carbodiimid mit Thiocarbamidsäureester-Gruppe: 34 N/50 mm
      Carbodiimid mit Harnstoff-Gruppe: 24 N/50 mm

   Des Weiteren wurde die Wärmestandfestigkeit bestimmt.
   Wärmestandfestigkeit (Ablaufstrecke bei 80°C in mm):
   Carbodiimid mit Thiocarbamidsäureester-Gruppe (Beispiel 1.1., erfindungsgemäß): 4 mm
   Carbodiimid mit Harnstoff-Gruppe (Beispiel 1.2., nicht erfindungsgemäß): 13 mm

## Patentansprüche

1. Thiocarbamide, enthaltend mindestens eine Carbodiimidgruppe und mindestens eine Thiocarbamidsäureestergruppe der Formel

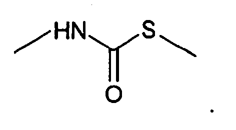

2. Thiocarbamide gemäß Anspruch 1, **dadurch gekennzeichnet, dass** sie zusätzlich hydrophile Gruppen enthalten.

3. Thiocarbamide gemäß Anspruch 1 oder 2, **dadurch gekennzeichnet, dass** die hydrophilen Gruppen ausgewählt sind aus ionischen Gruppen oder nichtionischen hydrophilen Gruppen.

4. Thiocarbamide gemäß einem der Ansprüche 1 bis 3, **dadurch gekennzeichnet, dass** es sich bei den nicht-ionischen Gruppen um Polyalkylenoxydgruppen handelt.

5. Thiocarbamide gemäß einem der Anprüche 1 bis 4, **dadurch gekennzeichnet, dass** die Verbindungen erhältlich sind durch Umsetzung von
a) Carbodiimide mit mindestens einer Isocyanatgruppe,
b) Mercaptoverbindungen mit mindestens einer Mercaptogruppe und
c) gegebenenfalls weiteren Verbindungen mit Isocyanatgruppen oder mit Isocyanat reaktiven Gruppen.

6. Thiocarbamide gemäß Anspruch 5, **dadurch gekennzeichnet, dass** sich die Carbodiimide a) von aliphatischen oder araliphatischen C4 bis C20 Polyisocyanaten ableiten.

7. Thiocarbamide gemäß Anspruch 5 oder 6, **dadurch gekennzeichnet, dass** die Mercaptoverbindungen b) neben mindestens einer Mercaptogruppe mindestens eine hydrophile Gruppe enthalten.

8. Thiocarbamide gemäß einem der Ansprüche 1 bis 7, **dadurch gekennzeichnet, dass** sie im Mittel 1 bis 20 Carbodiimidgruppen und 1 bis 4 Thiocarbamidsäureestergruppen enthalten.

9. Thiocarbamide gemäß einem der Ansprüche 1 bis 8, **dadurch gekennzeichnet, dass** sie im Mittel 0,01 bis 2 Mol hydrophile Gruppen pro 1 kg Thiocarbamid enthalten.

10. Verwendung von Thiocarbamiden gemäß einem der Ansprüche 1 bis 9 als Vernetzer für in Wasser dispergierte oder gelöste Polymere.

11. Wässrige Polymerdispersionen oder Polymerlösungen enthaltend 0,1 bis 50 Gew. Teile eines Thiocarbamids gemäß einem der Ansprüche 1 bis 9 auf 100 Gew. Teile Polymer.

12. Wässrige Polymerdispersionen oder Polymerlösungen gemäß Anspruch 11, **dadurch gekennzeichnet, dass** es sich bei dem dispergierten oder gelösten Polymer um ein Polyurethan oder um ein durch radikalische Polymerisation von ethylenisch ungesättigten Verbindungen (Monomere) erhältliches Polymer (kurz Polyaddukt) handelt.

13. Wässrige Polymerdispersionen oder Polymerlösungen gemäß Anspruch 11 oder 12, **dadurch gekennzeichnet, dass** das gelöste oder dispergierte Polymer einen Gehalt an Carbonsäuregruppen hat.

14. Verwendung der Polymerdispersionen oder-lösungen gemäß einem der Ansprüche 11 bis 13 als Klebstoff, Imprägnierungs- oder Beschichtungsmittel.

15. Verwendung der Polymerdispersionen oder -lösungen gemäß einem der Ansprüche 11 bis 13 als oder in hitzeaktivierbaren Klebstoffen.

16. Mit Polymerdispersionen oder -lösungen gemäß einem der Ansprüche 11 bis 13 beschichtete, imprägnierte oder verklebte Substrate.

17. Verwendung von Thiocarbamiden gemäß einem der Ansprüche 1 bis 9 als Stabilisator für ester- oder amidgruppenhaltige Polymere.

## Claims

1. A thiocarbamide comprising at least one carbodiimide group and at least one thiocarbamic ester group of the formula

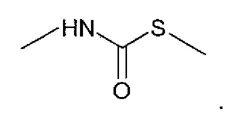

2. The thiocarbamide according to claim 1, further comprising hydrophilic groups.

3. The thiocarbamide according to claim 1 or 2, wherein the hydrophilic groups are selected from ionic groups or nonionic hydrophilic groups.

4. The thiocarbamide according to any one of clams 1 to 3, wherein the nonionic groups are polyalkylene oxide groups.

5. The thiocarbamide according to any one of claims 1 to 4, wherein the compounds are obtainable by reacting
a) carbodiimides having at least one isocyanate group,
b) mercapto compounds having at least one mercapto group, and
c) if appropriate, further compounds having isocyanate groups or isocyanate-reactive groups.

6. The thiocarbamide according to claim 5, wherein the carbodiimides a) derive from aliphatic or araliphatic C4 to C20 polyisocyanates.

7. The thiocarbamide according to claim 5 or 6, wherein the mercapto compounds b) comprise not only at least one mercapto group but also at least one hydrophilic group.

8. The thiocarbamide according to any one of claims 1 to 7, comprising on average from 1 to 20 carbodiimide groups and from 1 to 4 thiocarbamic ester groups.

9. The thiocarbamide according to any one of claims 1 to 8, comprising on average from 0.01 to 2 mol of hydrophilic groups per 1 kg of thiocarbamide.

10. The use of a thiocarbamide according to any one of claims 1 to 9 as crosslinker for polymers in solution or dispersion in water.

11. An aqueous polymer dispersion or polymer solution comprising from 0.1 to 50 parts by weight of a thiocarbamide according to any one of claims 1 to 9 per 100 parts by weight of polymer.

12. The aqueous polymer dispersion or polymer solution according to claim 11, wherein the dispersed or dissolved polymer is a polyurethane or a polymer obtainable by free-radical addition polymerization of ethylenically unsaturated compounds (monomers) (polyadduct for short).

13. The aqueous polymer dispersion or polymer solution according to claim 11 or 12, wherein the dissolved or dispersed polymer contains carboxylic acid groups.

14. The use of the polymer dispersion or solution according to any one of claims 11 to 13 as an adhesive, impregnant or coating material.

15. The use of the polymer dispersion or solution according to any one of claims 11 to 13 as or in heat-activable adhesives.

16. A substrate coated, impregnated or adhesively bonded with the polymer dispersion or solution according to any one of claims 11 to 13.

17. The use of a thiocarbamide according to any one of claims 1 to 9 as a stabilizer for polymers containing ester or amide groups.

## Revendications

1. Thiocarbamides contenant au moins un groupe carbodiimide et au moins un groupe ester thiocarbamique de la formule :

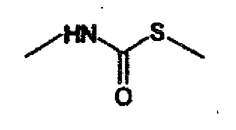

2. Thiocarbamides suivant la revendication 1, **caractérisés en ce qu'**ils contiennent en outre des groupes hydrophiles.

3. Thiocarbamides suivant la revendication 1 ou 2, **caractérisés en ce que** les groupes hydrophiles sont choisis parmi des groupes ioniques ou des groupes hydrophiles non ioniques.

4. Thiocarbamides suivant l'une quelconque des revendications 1 à 3, **caractérisés en ce que** les groupes non ioniques sont des groupes de poly(oxyde d'alkylène).

5. Thiocarbamides suivant l'une quelconque des revendications 1 à 4, **caractérisés en ce que** les composés peuvent être obtenus par réaction de :
a) carbodiimides comportant au moins un groupe isocyanate,
b) composés mercapto comportant au moins un groupe mercapto et
c) le cas échéant, d'autres composés comportant des groupes isocyanate ou des groupes réactifs aux isocyanates.

6. Thiocarbamides suivant la revendication 5, **caractérisés en ce que** les carbodiimides a) dérivent de polyisocyanates aliphatiques ou araliphatiques en C₄ à C₂₀.

7. Thiocarbamides suivant la revendication 5 ou 6, **caractérisés en ce que** les composés mercapto b) contiennent, outre au moins un groupe mercapto, au moins un groupe hydrophile.

8. Thiocarbamides suivant l'une quelconque des revendications 1 à 7, **caractérisés en ce qu'**ils contiennent en moyenne de 1 à 20 groupes carbodiimide et de 1 à 4 groupes ester thiocarbamique.

9. Thiocarbamides suivant l'une quelconque des revendications 1 à 8, **caractérisés en ce qu'**ils contiennent en moyenne de 0,01 à 2 moles de groupes hydrophiles par kg de thiocarbamide.

10. Utilisation de thiocarbamides suivant l'une quelconque des revendications 1 à 9 en tant que réticulants pour des polymères dispersés ou dissous dans de l'eau.

11. Dispersions ou solutions aqueuses de polymères, contenant de 0,1 à 50 parties en poids d'un thiocarbamide suivant l'une quelconque des revendications 1 à 9 pour 100 parties en poids de polymère.

12. Dispersions ou solutions aqueuses de polymères suivant la revendication 11, **caractérisées en ce que** le polymère dispersé ou dissous est un polyuréthanne ou un polymère qui peut être obtenu par polymérisation radicalaire de composés (monomères) éthyléniquement insaturés (en abrégé produit de polyaddition).

13. Dispersions ou solutions aqueuses de polymères suivant la revendication 11 ou 12, **caractérisées en ce que** le polymère dissous ou dispersé possède une certaine teneur en groupes acide carboxylique.

14. Utilisation des dispersions ou des solutions de polymères suivant l'une quelconque des revendications 11 à 13 en tant qu'adhésifs, agents d'imprégnation ou agents de revêtement.

15. Utilisation des dispersions ou des solutions de polymères suivant l'une quelconque des revendications 11 à 13 en tant que ou dans des adhésifs activables par la chaleur.

16. Substrats revêtus, imprégnés ou collés au moyen de dispersions ou de solutions de polymères suivant l'une quelconque des revendications 11 à 13.

17. Utilisation de thiocarbamides suivant l'une quelconque des revendications 1 à 9 en tant que stabilisants pour des polymères contenant des groupes ester ou amide.
